# EUROPEAN PATENT APPLICATION

(11) **EP 4 793 671 A1**
(43) Date of publication of application: **19.08.2026**
(21) Application number: 24877580.1
(22) Date of filing: 14.10.2024
(51) Int. Cl.: G01T 1/24, A61B 6/42, A61B 6/51, A61B 6/50, H10F 30/00, H10F 77/20, H10F 77/12, H10F 10/17

(54) **X-RAY DETECTOR**

(30) Priority: 13.10.2023 US 202363590092 P; 01.11.2023 KR 20230149160
(71) Applicant: Rayence Co., Ltd., Hwaseong-si, Gyeonggi-do 18449 (KR); Vatech Ewoo Holdings Co., Ltd., Hwaseong-si, Gyeonggi-do 18449 (KR); Qpix solutions Inc., Morrisville, NC 27560 (US)
(72) Inventor: YUN, Seung Man, Morrisville, North Carolina 27560 (US); LEE, Seung Hyeon, Morrisville, North Carolina 27560 (US); LEE, Won Jun, Morrisville, North Carolina 27560 (US); WILLIAMSON, Carter, Morrisville, North Carolina 27560 (US); HANY, Ibrahim, Morrisville, North Carolina 27560 (US)
(74) Representative: Lorenz & Kollegen
(86) International application number: PCT/KR2024/015518
(87) International publication number: WO 2025/080045

(57) **Abstract**

The present invention provides an X-ray detector comprising: first and second electrodes on a substrate; and a photo electric material layer formed between the first electrode and the second electrode and containing perovskite, wherein the perovskite has a cubic crystal structure, and the size of the perovskite crystals is 3-10 um.

## Description

### Technical Field

The present disclosure relates to an X-ray detector.

### Background Art

In recent years, digital detectors have become widely used for X-ray imaging.

X-ray detectors are divided into indirect-conversion and direct-conversion types. Indirect-conversion detectors have a scintillator that first converts X-rays into visible light, and then the visible light is converted to electrical signals. In direct conversion, on the other hand, a photoconductor that absorbs X-rays and directly creates electrical signals is used.

As a photoconductor, perovskite has attracted substantial attention. However, according to current research, there are limitations in applying perovskite as a photoconductor for X-ray detectors.

### Disclosure

### Technical Problem

Accordingly, the present disclosure has been made keeping in mind the above problems occurring in the related art, and the present disclosure is intended to provide a method for effectively using perovskite as a photoconductor for X-ray detectors.

### Technical Solution

In order to achieve the above objective, according to an aspect of the present disclosure, there is provided an X-ray detector including: first and second electrodes on a substrate; and a photoconductor layer provided between the first electrode and the second electrode and configured to contain perovskite, wherein the perovskite may have a cubic crystal structure, and a crystal size of the perovskite may be 3 µm to 10 µm.

Within the photoconductor layer, the crystal size of the perovskite gradually may increase as the distance from the substrate increases.

When the X-ray detector is used as a dental intraoral sensor or a mammography sensor, the thickness of the photoconductor layer may be 50 µm to 200 µm.

When the X-ray detector is used as a dental CBCT sensor or a general radiography sensor, the thickness of the photoconductor layer may be 200 µm to 700 µm.

When the X-ray detector is used as a sensor for high-energy equipment, such as a dental MDCT sensor, the thickness of the photoconductor layer may be 0.7 mm to 2 mm.

The perovskite may be CsPbBr3, Cs2AgBiBr6, MAPbI3, or MAPbBr3.

### Advantageous Effects

According to the present disclosure, in forming a photoconductor layer using perovskite, the crystal size of the perovskite is set to 3 µm to 10 µm, and within this size range, the crystal size of the perovskite gradually increases toward the top in the photoconductor layer, and the thickness of the photoconductor layer is set according to the purpose.

Therefore, the photoconductor layer formed of perovskite can have excellent electrical properties while ensuring sufficient adhesion to a substrate, and an X-ray detector provided with the photoconductor layer formed of perovskite can be applied to various X-ray machines.

### BRIEF DESCRIPTION OF THE DRAWINGS

The above and other objectives, features, and other advantages of the present disclosure will be more clearly understood from the following detailed description when taken in conjunction with the accompanying drawings, in which:
FIG. 1 is a schematic view of an X-ray detector according to an embodiment of the present disclosure;
FIG. 2 is a cross-sectional view schematically showing an X-ray detector according to an embodiment of the present disclosure; and
FIG. 3 is an enlarged cross-sectional view of a photoconductor layer according to an embodiment of the present disclosure.

### DETAILED DESCRIPTION

Hereinafter, embodiments of the present disclosure will be described in detail with reference to the drawings.

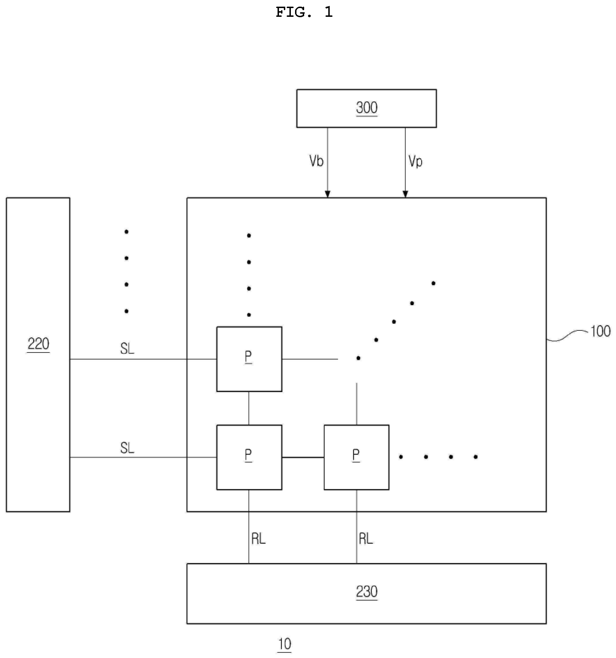

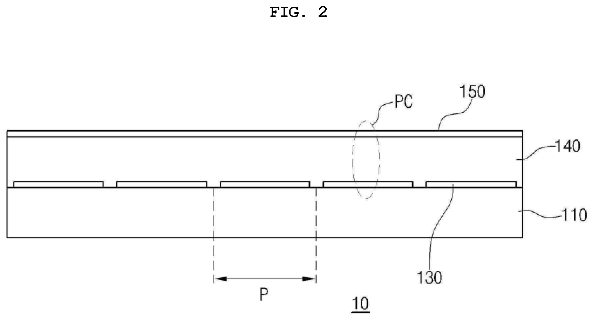

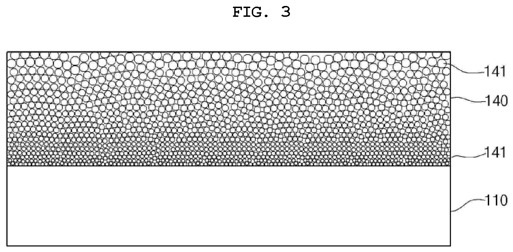

FIG. 1 is a schematic view of an X-ray detector according to an embodiment of the present disclosure. FIG. 2 is a cross-sectional view schematically showing an X-ray detector according to an embodiment of the present disclosure. FIG. 3 is an enlarged cross-sectional view of a photoconductor layer according to an embodiment of the present disclosure.

In FIG. 3, for convenience of explanation, first and second electrodes located below and above a photoconductor layer are omitted.

Referring to FIGS. 1 to 3, an X-ray detector 10 according to an embodiment of the present disclosure corresponds to a direct-conversion X-ray detector provided with a photoconductor layer 140.

The X-ray detector 10 may include a sensor panel 100, a driving circuit part for driving the sensor panel 100, and a power circuit 300 that supplies a driving voltage (or power voltage) to drive the X-ray detector 10.

The sensor panel 100 may be a direct-conversion sensor panel 100 that directly converts incident X-rays into electrical signals.

Although not specifically shown, the sensor panel 100 may include: an active area (display area), which is the area that actually receives and detects X-rays; and a non-active area (non-display area) located outside the active area.

In the active area, a pixel array composed of a plurality of pixels P is disposed on a substrate 110, and the pixels P may be arranged in a matrix form along a plurality of row lines and a plurality of column lines.

On the substrate 110, a plurality of scan lines (or gate lines) SL extending along the row lines and a plurality of readout lines (or data lines) RL extending along the column lines may be disposed. The scan lines SL and the readout lines RL may be connected to corresponding pixels P.

Meanwhile, in the embodiment, the substrate 110 may be composed of, but is not limited to, a CMOS substrate, a glass substrate, or a plastic substrate having flexible properties.

The driving circuit part for driving the sensor panel 100 may include a scan circuit 220 and a readout circuit 230.

In this case, the scan circuit 220 sequentially scans the scan lines SL and applies a scan signal of a turn-on level. Accordingly, individual row line is sequentially selected, and data, which is an electrical signal stored in the pixel P located in the selected row line, may be output to the corresponding readout line RL. Then, the readout circuit 230 may receive the data stored in the pixel P through the readout line RL.

Each pixel P of the sensor panel 100 may be provided with a photoconductive element PC that detects X-rays and generates a corresponding electrical signal.

In this regard, the photoconductive element PC may include: a first electrode (or pixel electrode) 130, which is a lower electrode provided on the substrate 110; a second electrode (or common electrode) 150, which is an upper electrode located above the first electrode 130; and the photoconductor layer 140 interposed between the first electrode 130 and the second electrode 150.

The first electrode 130 may be provided in a patterned form on a pixel-by-pixel basis corresponding to each pixel P.

The photoconductor layer 140 provided on the first electrode 130 may be formed, for example, continuously along the pixels P substantially arranged in the active area. In other words, the photoconductor layer 140 may be formed corresponding to the pixels P arranged in the active area.

As a photoconductor forming the photoconductor layer 140, perovskite may be used.

The perovskite is a material with a crystal structure following the formula ABX3, where A denotes a monovalent cation, B denotes a metal cation, and X may denote a halogen anion.

The perovskite may be, but is not limited to, CsPbBr3, Cs2AgBiBr6, MAPbI3, or MAPbBr3.

Meanwhile, the photoconductor layer 140 of perovskite may be formed, for example, by a solution method. The photoconductor layer 140 of perovskite may be formed through a process of applying and curing a solution (or paste) containing a perovskite powder and a solvent onto the substrate 110 on which the first electrode 130 is provided.

The second electrode 150 provided on the photoconductor layer 140 may be formed, for example, continuously along the pixels P substantially arranged in the active area. In other words, the second electrode 150 may be formed corresponding to the pixels P arranged in the active area. A bias voltage (i.e., reverse voltage) may be applied to the second electrode 150.

Meanwhile, in the embodiment, a perovskite crystal 141 constituting the photoconductor layer 140 may be formed into a cubic structure, and thus may have excellent electrical properties.

In this case, the size (or diameter) of the perovskite crystal 141 is preferably, for example, approximately 3 µm to 10 µm.

As the size of the perovskite crystal 141 increases, the porosity increases, which may reduce the adhesion of the photoconductor layer 140 to the substrate 110. In particular, the photoconductor layer 140 of the perovskite has a large difference in thermal expansion rate from the substrate 110 due to the characteristics thereof, and thus the photoconductor layer 140 may be detached (or peeled) from the substrate 110 depending on the temperature. Considering this, it is desirable that the size of the perovskite crystal 141 be 10 µm or less.

In addition, when the size of the perovskite crystal 141 is smaller than 3 µm, it becomes difficult to maintain the cubic structure. Considering this, it is preferable that the size of the perovskite crystal 141 be 3 µm or larger.

Thus, considering the porosity and cubic crystal structure within the photoconductor layer 140, the size of the perovskite crystal 141 may be set to approximately 3 µm to 10 µm.

In addition, to realize the perovskite crystal 141 of the above size, the size of the perovskite powder mixed in the solvent before application may be approximately 3 µm to 7 µm.

Meanwhile, as shown in FIG. 2, within the photoconductor layer 140, the perovskite crystal 141 may be configured to gradually increase in size going upward (i.e., away from the substrate 110).

That is, the perovskite crystal 141 formed at the lower part of the photoconductor layer 140 close to the substrate 110 may be relatively small in size, whereas the perovskite crystal 141 formed at the upper part of the photoconductor layer 140 away from the substrate 110 may be relatively large in size.

As such, in the part of the photoconductor layer 140 close to the substrate 110, the small-sized perovskite crystals 141 with relatively high adhesion characteristics are formed, so that the adhesion of the photoconductor layer 140 to the substrate 110 may be effectively ensured.

Regarding implementing a structure in which the size of the crystal 141 gradually increases toward the top in the photoconductor layer 140 as described above: in the process of curing after applying the mixed solution of perovskite powder and solvent, heat is applied to the substrate 110 (more specifically, heat is applied from the bottom of the substrate 110) to first evaporate the solvent close to the substrate 110, which allows the size of the perovskite crystal 141 to gradually increase toward the top.

Regarding curing conditions for the above curing process, the curing process may be performed on the applied perovskite solution at a temperature of approximately room temperature to 90 degrees for approximately 10 to 20 hours, for example.

Meanwhile, the thickness of the photoconductor layer 140 containing the perovskite crystal 141 may be appropriately set in consideration of an X-ray machine (or purpose) to which the X-ray detector 10 is applied.

In this regard, for example: when the X-ray detector 10 is used as a dental intraoral sensor or a mammography sensor, the thickness of the photoconductor layer 140 may be approximately 50 µm to 200 µm.

When the X-ray detector 10 is used as a dental CBCT sensor or a general radiography sensor, the thickness of the photoconductor layer 140 may be approximately 200 µm to 700 µm.

In addition, when the X-ray detector 10 is used as a sensor for high-energy equipment, such as a dental MDCT sensor, the thickness of the photoconductor layer 140 may be approximately 0.7 mm to 2 mm.

As described above, in the embodiment of the present disclosure, in forming the photoconductor layer 140 using perovskite, the size of the perovskite crystal 141 is set to 3 µm to 10 µm, and within this size range, the size of the perovskite crystal 141 gradually increases toward the top in the photoconductor layer 140, and the thickness of the photoconductor layer 140 is set according to the purpose.

Therefore, the photoconductor layer 140 formed of perovskite may have excellent electrical properties while ensuring sufficient adhesion to the substrate 110, and the X-ray detector 10 provided with the photoconductor layer 140 formed of perovskite may be applied to various X-ray machines.

The above-described embodiment of the present disclosure is an example of the present disclosure, and free modification is possible within the scope included in the spirit of the present disclosure. Accordingly, the present disclosure includes modifications of the present disclosure within the scope of the appended claims and the equivalents thereof.

## Claims

1. An X-ray detector comprising:
first and second electrodes on a substrate; and
a photoconductor layer provided between the first electrode and the second electrode and configured to contain perovskite,
wherein the perovskite has a cubic crystal structure, and a crystal size of the perovskite is 3 µm to 10 µm.

2. The X-ray detector of claim 1, wherein within the photoconductor layer, the crystal size of the perovskite gradually increases as a distance from the substrate increases.

3. The X-ray detector of claim 1 or 2, wherein when the X-ray detector is used as a dental intraoral sensor or a mammography sensor, a thickness of the photoconductor layer is 50 µm to 200 µm.

4. The X-ray detector of claim 1 or 2, wherein when the X-ray detector is used as a dental CBCT sensor or a general radiography sensor, a thickness of the photoconductor layer is 200 µm to 700 µm.

5. The X-ray detector of claim 1 or 2, wherein when the X-ray detector is used as a sensor for high-energy equipment, such as a dental MDCT sensor, a thickness of the photoconductor layer is 0.7 mm to 2 mm.

6. The X-ray detector of claim 1 or 2, wherein the perovskite is CsPbBr3, Cs2AgBiBr6, MAPbI3, or MAPbBr3.
